# EUROPEAN PATENT SPECIFICATION

(11) **EP 4 669 299 B1**
(45) Date of publication and mention of the grant of the patent: **09.12.2026**
(21) Application number: 24711465.5
(22) Date of filing: 22.02.2024
(51) Int. Cl.: A61K 8/36, A61K 8/67, A61K 8/9783, A61P 17/14, A61Q 7/00

(54) **COMPOSITION FOR IMPROVING THE AESTHETIC APPEARANCE OF HAIR AND SKIN APPENDAGES**
ZUSAMMENSETZUNG ZUR VERBESSERUNG DES ÄSTHETISCHEN ERSCHEINUNGSBILDES VON HAAR UND HAUTANHANGSGEBILDEN
COMPOSITION POUR AMÉLIORER L'ASPECT ESTHÉTIQUE DES PHANÈRES

(30) Priority: 24.02.2023 IT 202300003327
(43) Date of publication of application: 31.12.2025
(73) Proprietor: Giuliani S.p.A., 20129 Milano (IT)
(72) Inventor: GIULIANI, Giammaria, 6926 MONTAGNOLA (CH); RINALDI, Fabio, 20122 MILANO (IT); PINTO, Daniela, 20151 MILANO (IT); MARZANI, Barbara, 27020 Carbonara Al Ticino (IT)
(74) Representative: Cattaneo, Elisabetta
(86) International application number: PCT/EP2024/054501
(87) International publication number: WO 2024/175703

(56) References cited:
- EP-B2- 0 289 639
- WO-A1-2007/086327
- WO-A2-00/03749
- FR-A1- 2 550 448
- FR-A1- 2 854 066
- JP-A- 2006 290 767
- KR-A- 20220 066 595
- US-A1- 2008 069 791

## Description

### FIELD OF THE INVENTION

The present invention relates to a composition for improving the aesthetic appearance of hair and skin appendages.

The present invention originates in the sector of products for the care and treatment of keratin structures, preferably hair and skin appendages.

In particular, a composition containing a synergistic combination of biologically active ingredients, that inhibit the glycogen phosphorylase enzyme, increase the glycogen metabolism of the hair follicle, stimulating hair growth and improving their aesthetic appearance, is provided herein.

### STATE OF THE ART

The life cycle of the hair bulb includes a growth phase known as anagen, a regression phase known as catagen, and a resting phase known as telogen.

In the anagen phase, the dermal papilla generates chemical signals that activate and instruct the bulb stem cells which, by migrating to the base of the follicle, form the hair matrix. With this "migration" the bulb stem cells create a "path" of cells that will give rise to the external root sheath or ORS.

In response to further signals from the dermal papilla, matrix cells, which are derived from stem cells, proliferate and begin the differentiation process, moving upward to form the shaft and inner sheath of the hair follicle.

The onset of the catagen phase is characterized by the end of cell proliferation and apoptosis of matrix cells. During catagen, the dermal papilla migrates towards the lower part of the bulb. This close proximity of the papilla to the bulb is believed to be essential for starting another hair production cycle. This allows for the interaction/activation of resting bulb cells and a new hair and skin appendages growth cycle.

After the catagen/telogen transition, some bulb cells migrate to meet the papilla, generating the hair germ.

Telogen hair contains a cell population at its base, which is effectively called the hair germ, located near the dermal papilla. The hair germ is activated to proliferate towards the end of the telogen phase, even before the bulb, to form the matrix of the new bulb, surrounding the papilla.

In order to maintain high proliferation rates, hair fiber production, and hair cycle-associated remodeling, the hair follicle (HF) requires energy and metabolite expenditure.

Human hair follicles have a particularly pronounced demand of precursors for energy and biosynthesis in the anagen phase, which is mainly satisfied by aerobic glycolysis, as highlighted by Purba TS, et al. in the publication "Characterisation of cell cycle arrest and terminal differentiation in a maximally proliferative human epithelial tissue: Lessons from the human hair follicle matrix." Eur. J. Cell Biol. 2017;96:632-641. doi: 10.1016/j.ejcb.2017.03.011.

From the publications by Vidali S, Knuever J, Lerchner J, Giesen M, Bíró T. Hypothalamic-pituitary-thyroid axis hormones stimulate mitochondrial function and biogenesis in human hair follicles. J. Invest. Dermatol. 2014;134:33-42. doi: 10.1038/jid.2013.286.; Lemasters JJ, et al. Compartmentation of mitochondrial and oxidative metabolism in growing hair follicles: A ring of fire. J. Invest. Dermatol. 2017;137:1434-1444. doi: 10.1016/j.jid.2017.02.983, it is clear that, despite the high number of mitochondria present, hair follicles preferentially acquire the energy necessary for their proliferation from aerobic glycolysis. This need is confirmed by the publication by Williams R, Philpott MP, Kealey T. Metabolism of freshly isolated human hair follicles capable of hair elongation: A glutaminolytic, aerobic glycolytic tissue. J Investig. Dermatol. 1993;100:834-840. doi: 10.1111/1523-1747.ep12476744).

The importance of aerobic glycolysis for the hair follicle function was also demonstrated in animal models through studies on the crucial role played by the enzyme lactate dehydrogenase and the metabolism of glucose into lactate in the activation of HF epithelial stem cells (HFSC), as illustrated in Flores A, et al. Lactate dehydrogenase activity drives hair follicle stem cell activation. Nat. Cell Biol. 2017;19:1017-1026. doi: 10.1038/ncb3575.

Hair follicles contain high levels of glycogen and glucose polymers, the main energy storage site in the human body. Glycogen, a glucose polysaccharide, is the main form of energy storage in mammals, and is also present at high levels in the follicle during the anagen growth phase (Figlak K, Williams G, Bertolini M, Paus R, Philpott MP. Human hair follicles operate an internal Cori cycle and modulate their growth via glycogen phosphorylase. Sci Rep. 2021 Oct 21;11(1):20761. doi: 10.1038/s41598-021-99652-8. PMID: 34675331; PMCID: PMC8531296.). These glucose levels decrease during the catagen growth phase, and glycogen is completely absent during the telogen phase.

Human hair follicles have the ability to synthesize glycogen from lactic acid through their own internal Cori cycle.

The follicle growth phase requires a great energy expenditure; for this reason, it is important to stimulate maximum gain/storage of energy by the follicle. *In vitro* studies on follicles demonstrated that inhibition of the key enzyme involved in energy storage through glycogen, *i.e.* glycogen phosphorylase (PYGL), increases its elongation and prolongs its anagen phase, thus delaying the transition into catagen (Figlak K, Williams G, Bertolini M, Paus R, Philpott MP. Human hair follicles operate an internal Cori cycle and modulate their growth via glycogen phosphorylase. Sci Rep. 2021 Oct 21;11(1):20761. doi: 10.1038/s41598- 021-99652-8. PMID: 34675331; PMCID: PMC8531296).

FR-A-2854066, KR-A-20220066595, WO2007086327, EP-A289639 and FR-A-2550448 disclose the positive effect of riboflavin on hair growth and hair loss. JP-A-2006290767, US- A-2008069791 and US-A6124362 disclose the positive effect of maslinic acid and olive extract on hair growth and hairloss.

One of the objects of the present invention consists in providing a composition in which biologically active ingredients, which intervene in the hair follicles energy metabolism by modulating and promoting their physiological growth thus improving their external appearance, are combined.

Another object of the invention consists in providing a composition that promotes glycogen metabolism in hair follicles, which can be formulated both as a dietary supplement for oral administration and as a topical formulation for application to areas of the body where stimulation of a keratin structure growth is desired, in particular hair and skin appendages.

Another object of the invention consists in providing a composition suitable for cosmetic use in improving the aesthetic appearance of the keratin structures of the human body, as well as for therapeutic use in preventing or treating hair and skin appendages growth disorders.

### SUMMARY OF THE INVENTION

In general terms, the present invention provides new uses in the cosmetic and trichology fields of a combination of biologically active ingredients.

According to some aspects of the invention, the inventors have found that the combination of selected biologically active components, which inhibit the activity of glycogen phosphorylase (PYGL), promotes the energy content and glycogen metabolism of the follicle, thus stimulating the growth of keratin structures, in particular hair and skin appendages in a mammal, preferably the hair of a human being.

In the present invention, when the term "skin appendages" is used, it is intended to comprise beard, eyelashes, eyebrows and nails.

According to a first aspect, the invention relates to the non-therapeutic, cosmetic use of a composition comprising a combination of maslinic acid and riboflavin, and a physiologically acceptable carrier for improving an aesthetic appearance of a keratin structure of a mammal selected from hair and skin appendages, in particular the hair of a human being.

Advantageously and preferably, a specific object of the invention is the non-therapeutic, cosmetic use of a composition comprising maslinic acid and riboflavin in promoting hair growth and/or preventing and/or reducing hair loss in a mammal, in particular a human being.

In accordance with certain specific aspects of the invention, the hair loss reduction activity of the composition described herein affects a population of individuals who are affected by a greater than physiological hair loss and which can cause thinning on the scalp.

In accordance with other aspects, the combination composition described herein finds application in the cosmetic field in improving an external aspect of hair and skin appendages, such as for example their volume, their shine and/or their compactness, their light-reflecting capacity and the liveliness of their color. According to certain embodiments, the composition for cosmetic use is selected from a topical composition, and a dietary supplement , which can be introduced into the dietary regime of an individual who wishes to improve the external appearance of hair and skin appendages.

According to another aspect, the invention relates to a composition comprising a combination of maslinic acid and riboflavin for use in the treatment of a disease or disorder in the growth of a keratin structure selected from hair and skin appendages, in particular hair.

For example, the composition for the therapeutic-trichological uses described herein is suitable for treating a disorder that causes or contributes to loss of a keratin structure, for example hair, and skin appendages.

Advantageously, it was found that the composition described herein, when administered topically or systemically to a subject suffering from thinning or loss of hair or skin appendages, causes progressive thickening and improvement of the keratin structure that makes it up, preferably, in the case of hair, thickening of the scalp.

For example, the composition including maslinic acid and riboflavin for the therapeutic uses described herein is indicated in the treatment of alopecia, one of the most widespread disorders in the growth of keratin structures, in particular of hair.

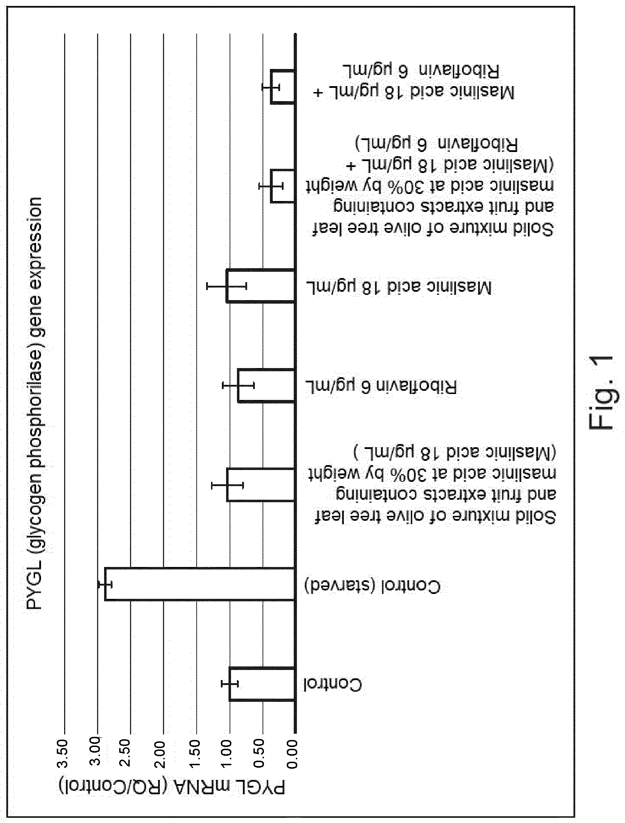

In particular, the composition finds application in the treatment of androgenetic alopecia (AGA). In individuals affected by androgenetic alopecia (AGA), follicles that are formed at the beginning of the new anagen phase decrease in size over time (miniaturization), progressively leading to the formation of hair smaller than the previous one. The result is the formation of a microscopic hair. This situation can be overcome by administering or applying locally the combination composition described herein, since its use increases the duration of the hair anagen phase. The composition described herein is also suitable for the treatment of forms of alopecia other than androgenetic alopecia, for example telogen effluvium, alopecia areata, ringworm, cicatricial alopecia, and also hair loss due to cosmetic overuse. Some aspects of the present invention are illustrated with reference to the accompanying Figure 1.

### DESCRIPTION OF THE FIGURE

The accompanying Figure 1 shows bar graphs relating to glycogen phosphorylase (PYGL) gene expression by qRT-PCR of Example 3, of the following ingredients: mixture of olive tree leaf and fruit extracts containing maslinic acid at 30% by weight (containing 18 µg/mL maslinic acid); 6 µg/mL riboflavin; 18 µg/mL maslinic acid; mixture of extracts from olive tree leaf and fruit containing maslinic acid at 30% by weight (18 µg/mL maslinic acid) and 6 µg/mL riboflavin (embodiment of the invention); mixture of 18 µg/mL maslinic acid and 6µg/mL riboflavin.

The bar graphs highlight how both the solid mixture of olive tree leaf and fruit extracts containing maslinic acid at 30% by weight and riboflavin, and maslinic acid in combination (mixture) with riboflavin, exert a synergistic and significantly (p<0.005) higher inhibition effect of PYGL gene expression, when compared to the positive control and individual components.

### DETAILED DESCRIPTION OF THE INVENTION

According to some aspects of the invention, the inventors have found that the combination of maslinic acid with riboflavin promotes cellular glycogen metabolism in hair follicles by stimulating the growth of keratin structures, in particular hair and skin appendages in mammals, preferably hair in human beings.

Therefore, an object of the invention is the use of a composition comprising maslinic acid and riboflavin, and a physiologically acceptable excipient as defined in the attached claim 1.

In accordance with this aspect, the composition described herein finds therefore application in improving an aesthetic appearance or an external characteristic of a keratin structure selected from hair and skin appendages, in particular of hair.

In the present invention, when the term "skin appendages" is used, it is intended to comprise hair, beard, eyelashes, eyebrows and nails.

In the context of the present invention, the term keratin structure means hair and skin appendages such as, preferably, beard, eyelashes, eyebrows and nails in a mammal which is a human being, or preferably the hair in the case of a mammal which is an animal being, for example a dog or cat or another pet or companion animal.

According to the invention, the composition according to any one of the embodiments described herein is useful in the cosmetic field for improving an aesthetic appearance of the hair and skin appendages such as, for example, hair pigmentation.

Typically, the composition comprising maslinic acid and riboflavin stimulates the physiological hair growth and hair thickening, improving the external appearance thereof.

This effect is attributable to an increase in the duration of the anagen phase of the hair follicle following the inhibitory effect of the combination composition on the expression of glycogen phosphorylase.

In accordance with a second aspect of the invention, a composition is provided comprising a combination of maslinic acid and riboflavin for use in the treatment of a disorder in the growth of a keratin structure selected from hair and skin appendages of a mammal. Preferably, the composition comprising a combination of maslinic acid and riboflavin is for use in the treatment of a hair growth disorder in a human being.

Hair growth disorders or conditions that can be treated according to this use comprise alopecia and *telogen effluvium,* disorders that affect male and female subjects. The composition is also suitable for the treatment or prevention of female patterns, baldness, or androgenetic alopecia.

The composition according to the invention, regardless of the cosmetic and/or therapeutic uses, comprises maslinic acid and riboflavin as biologically active ingredients or components.

This combination results in a synergistic inhibition action of glycogen phosphorylase. One of the components of the composition of the invention is maslinic acid, also known as crategolic acid or (2α,3β)-2,3-dihydroxyolean-12-en-28-oic acid (IUPAC), CAS N. 4373-41-5, having the following chemical structure:

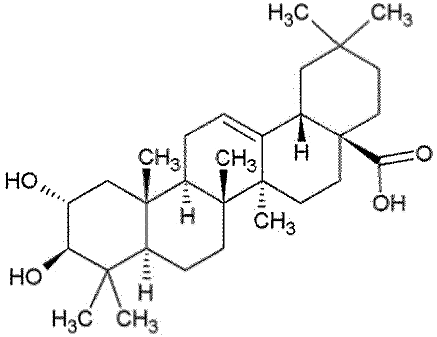

Maslinic acid belongs to the group of pentacyclic triterpenes, known as oleananes. This compound is widely distributed in the plant kingdom, and can be derived from wax present on olives skin and/or from dry olive pomace oil, a by-product of olive oil extraction. Typically, it is a by-product of olive oil extraction from the fruits of the tree, particularly from *Olea europaea.*

In accordance with some embodiments, maslinic acid is of natural origin and is contained in a solid mixture extracted from olive tree leaves and/or fruits (Olea *Europea*)*.*

Maslinic acid may be present in the composition in the form of a plant extract obtained from olive tree leaves and/or olives, in particular from their peel.

In accordance with some embodiments, maslinic acid is comprised in a composition in solid form, for example in the form of a tablet, in an amount in the range from 0.18 mg to 180 mg, preferably from 3 to 100 mg, for example 18 mg; in the composition in liquid form maslinic acid is comprised in a range from 6 µg/mL to 6 mg/mL, preferably from 100 µg/mL to 3 mg/mL, for example 0.06 g/100 mL.

Within the scope of the invention, a suitable plant extract comprising maslinic acid can be obtained from olive tree fruits, in particular from the *Olea europaea* tree. For the uses according to the invention, the extract is preferably obtained from olive tree fruits, i.e. olives.

In one embodiment, maslinic acid can be obtained from a solid mixture extracted from olive tree leaf and fruits.

Preferably an oil extracted from olive tree fruits and/or leaves may comprise maslinic acid in an amount greater than or equal to 20% w/w, more preferably in an amount in the range from 25 to 45%, even more preferably from 30 to 35% and advantageously 30% w/w.

The plant extract comprising maslinic acid can be obtained by extraction from olives using a physiologically acceptable solvent as an extraction medium.

The term "physiologically acceptable solvent" means a solvent that does not produce significant adverse reactions when introduced into the human body or applied to the human body. A suitable solvent for obtaining the plant extract is a physiologically acceptable liquid, in which the biologically active components of the selected plant are soluble and in which they do not undergo an alteration that deprives them of activity.

In some embodiments, the physiologically acceptable solvent is a protic polar solvent, preferably selected from the group consisting of water, acetic acid, ethanol, n-butanol ethyl acetate, n-butanol, isopropanol, n-propanol, and mixtures thereof. Preferably the extraction solvent is a polar protic solvent selected from the group consisting of water, ethanol, ethyl acetate and mixtures thereof, more preferably it is a mixture of water and ethyl acetate.

To obtain the plant extract from the olive tree, well-known extraction techniques such as, for example, solid-liquid extraction, can be used to separate/extract one or more biologically active components from the vegetal tissues of the tree.

In certain embodiments, the extraction of one or more biologically active components occurs by extraction of a portion or plant matrix of *Olea europaea* in a suitable solvent.

In accordance with a preferred and advantageous embodiment, the production process of a suitable extract of maslinic acid from olive tree fruits comprises the following steps:
a) extracting from olives using a protic polar solvent, preferably a mixture of water and ethyl acetate;
b) drying the extract obtained in step a) with removal of the solvent. Advantageously, between the two steps a) and b) a purification step of the olive extraction product is provided.

For example, a suitable extract can be obtained by immersing or macerating a portion of olives in a mixture of water and ethyl acetate, for a time suitable to enrich the solvent with one or more biologically active components. Under these conditions, the extraction of the biologically active components from the vegetal tissues of the selected plant occurs essentially by diffusion and/or osmosis. The maceration time of the plant portions in the solvent is variable, for example from 1 to 48 hours. The material obtained from the extraction is purified and subsequently dried.

In accordance with certain embodiments, the preparation of a suitable extract from olives, in particular from the peel, comprises the following steps:
i) crushing olives or olive peels,
ii) adding to the crushed product of step i) an extraction solvent, for example a water-ethyl acetate mixture, to obtain a mixture having a solid/hydroalcoholic solvent ratio in the range from about 1:10 to about 1:50 w/w,
iii) macerating the mixture of step ii),
iv) extracting the biologically active components,
v) filtrating said biologically active components,
vi) concentrating the filtrate, for example, at reduced pressure by evaporation of the hydroalcoholic solvent, obtaining an extract,
vii) drying the extract thus obtained.

In accordance with another embodiment, the extraction method from olives comprises the following steps:
A) crushing olives, obtaining a powder;
B) transferring the powder obtained into a suitable percolator;
C) performing a percolation, for example with an amount of extraction solvent so as to have a solid/solvent weight ratio from about 1:20 to about 1:100
D) recirculating part of the leachate until the material to be extracted is exhausted, obtaining a vegetable bed,
E) performing the squeezing of the extracted vegetable bed to recover all the extraction solvent
F) filtrating the leachate
G) concentrating the filtrate, for example at reduced pressure, by evaporation of the solvent.

According to some embodiments, in the final step of solvent removal by evaporation, a solid support is added, for example a starch or a maltodextrin, to obtain the extract in the form of a dry powder.

The extract obtained from olives can be fluid, soft or dry.

It is possible to prepare olive extracts of different polarities.

In certain embodiments, the extraction is accomplished using a solvent to vegetal matrix weight ratio in the range from 1:10 to 10:1.

Preferably the extract obtained from olive tree fruits and/or leaves comprises maslinic acid and/or oleanolic acid, and more preferably in said extract the sum of the amounts of maslinic acid and oleanolic acid is in the range from 10 to 80%, from 30 to 80% and even more preferably it is from 75% to 80% by weight.

In the composition described herein, maslinic acid is combined with riboflavin, known as vitamin B2.

Riboflavin is a water-soluble biologically active substance naturally present in plants and is a crucial micronutrient that is a precursor to coenzymes flavin mononucleotide and flavin adenine dinucleotide, being required for biochemical reactions in all living cells.

Riboflavin is produced by all plants and most microorganisms, and it is essential for the growth and reproduction of human and animal cells.

Riboflavin performs its biochemical function as a precursor for the coenzymes, flavin adenine dinucleotide (FAD) and flavin mononucleotide (FMN), involved in numerous redox reactions in living organisms.

These flavocoenzymes participate in the metabolism of carbohydrates, lipids, ketone bodies and proteins, from which living organisms derive most of their energy. Riboflavin and its coenzyme forms, FMN and FAD, have an inhibitory action on glycogen phosphorylase (PYGL). The inventors have now observed that this action of riboflavin is unexpectedly increased when it is combined with maslinic acid.

In accordance with some embodiments, riboflavin is comprised in the composition described herein in an amount in the range from 0.5 mg to 72 mg, preferably from 2 to 18 mg, for example 6 mg in the form of a tablet.

Preferably, the ratio between riboflavin and maslinic acid is in the range from 0.5 - 1.5 of riboflavin to 3 of maslinic acid, for example it is equal to 1:3.

In one embodiment the composition described herein contains oleanolic acid, for example from 0.1 to 10%, preferably from 0.5 to 5% by weight. Preferably, oleanolic acid is present in the composition of the invention in the form of an extract. More preferably, oleanolic acid is contained in the extract comprising also maslinic acid. In this last embodiment, the olive tree extract comprises both maslinic acid and oleanolic acid, which are therefore present in the composition of the invention as a single extract containing them.

The composition described herein for cosmetic or therapeutic-trichological uses is suitable for local or systemic administration.

In accordance with some embodiments, the composition for oral administration is or is contained in a functional food, a dietary supplement, or a nutritional product. Functional food means any food or modified food ingredient that can provide a benefit or protection against an ailment or physiological condition, in addition to the traditional nutrients contained therein.

Regardless of the route of administration, the cosmetic composition increases the viability of the follicle cells during the anagen phase and activates the quiescent cells of the keratin structure, preferably of the scalp, stimulating the metabolism of the follicles and the growth of new hair or skin appendages.

In certain embodiments, the composition of the invention may further comprise one or more additional ingredients having trichological activity.

In some embodiments, compositions for both cosmetic and therapeutic uses further comprise additional ingredients such as vitamins, for example vitamin A, vitamin C, additional B vitamins, niacin, and mixtures thereof.

According to some embodiments, the composition of the invention further comprises an amino acid, in particular a sulfur-containing amino acid, such as L-cysteine or L-methionine, preferably for the preparation of formulations for oral administration. According to some embodiments, the composition of the invention also comprises one or more micronutrients and/or minerals or micronutrients such as Mg, Zn, Ca, Fe, Cr, Se and others, preferably for the preparation of formulations for oral administration.

In some embodiments, the composition may further comprise additional substances such as folic acid, calcium pantothenate, mucopolysaccharides such as hyaluronic acid, or soy derivatives such as soy isoflavones.

In some embodiments, the composition of the invention for both cosmetic and medical uses comprises a physiologically and/or pharmaceutically acceptable carrier, diluent or excipient.

Typically, the physiologically acceptable carrier of the composition of the invention is an excipient, carrier or diluent suitable for topical application and/or systemic administration. The physiologically and pharmacologically acceptable carriers can be the same carriers.

In the context of the present document, the term "carrier" refers to an excipient, carrier, diluent, or adjuvant that may be present in the composition of the invention. Any carrier and/or excipient suitable for the desired preparation form for administration is contemplated in the uses of the plant extract or active ingredients present described herein.

In the context of the present description, the term "combination" is intended to mean that the substances maslinic acid and riboflavin are both present in the composition, for example as a mixture, without however the two substances interacting with each other from a chemical point of view and/or with the formation of chemical bonds between them.

In the context of the present document, and as indicated above, preferably the term keratin structure is intended to mean hair, beard, eyelashes, eyebrows in a human being or hair in the case of a mammal, for example a dog or cat or another pet or companion animal.

Typically, a suitable carrier is a physiologically, edible, or pharmaceutically acceptable carrier.

Typically, the compositions for oral administration may contain one or more edible carriers.

The compositions of the present invention comprise any composition made by mixing maslinic acid and riboflavin and a physiologically acceptable carrier. Such compositions are suitable for nutritional, pharmaceutical or dietary use in a mammal, in particular human beings.

The carrier may have a wide variety of suitable forms based on the desired preparation form for both oral and local administration.

Accordingly, the physiologically and/or pharmaceutically acceptable carrier, diluent or excipient may be selected based on the route of administration for which the composition is intended.

The compositions for oral administration can be in solid or liquid form.

Typical compositions in solid form comprise tablets, capsules, powders, granules, pills. If desired, the tablets may be coated with a suitable polymeric agent or an agent to achieve sustained/extended release by conventional techniques.

Exemplary excipients used in solid forms include cellulose derivatives such as hydroxymethyl cellulose, hydroxypropyl methylcellulose, methylcellulose, hydroxypropyl cellulose, hydroxyethyl cellulose, carboxyethyl cellulose, ethylhydroxyethyl cellulose, cellulose acetate butyrate, cellulose acetate phthalate, and mixtures thereof.

Further examples of suitable excipients include polymers of the lactam class, typically pyrrolidone and derivatives thereof, such as polyvinylpyrrolidone, polyvinylpolypyrrolidone and mixtures thereof, inorganic salts such as calcium or dicalcium phosphate, lubricants such as magnesium stearate, triacylglycerols and mixtures thereof.

Tablets, pills, capsules, and the like may also contain a binder such as gum tragacanth, acacia, corn starch or gelatin; excipients such as dicalcium phosphate; a disintegrating agent such as corn starch, potato starch, alginic acid; a lubricant such as magnesium stearate; and a sweetening agent such as sucrose, lactose, saccharin, or other sweeteners. When a unit dosage form is a capsule, in addition to materials of the type indicated above, it may contain a liquid carrier such as a fatty oil.

In certain embodiments, the composition of the invention further comprises one or more additional components, such as additives, fillers, stabilizers, emulsifiers, structurants, film-forming agents, plasticizers, wetting agents, and thickeners. Various other materials may be present as coatings or to modify the physical shape of the dosage unit. For example, tablets may be coated with shellac, sugar, or both. In addition to the active ingredient, a syrup or elixir may contain sucrose as a sweetening agent, methyl and propylparaben as preservatives, a coloring agent and a flavoring agent such as cherry or orange flavor. To prevent rupture during transit through the upper portion of the gastrointestinal tract, the composition may be an enteric-coated formulation.

Typical compositions in liquid form for oral administration comprise solutions, suspensions, syrups, emulsions, gels.

In these forms of administration, suitable carriers include water, glycols, or oil-in-water or water-in-oil emulsions containing the biologically active components dispersed therein.

In some embodiments, the route of administration of the composition of the invention is topical. The compositions for topical administration can be in solid, semi-liquid or liquid form. Typically, the topical composition is applied to the skin, and especially to the scalp in an effective amount.

For example, for cosmetic uses, a cosmetically active amount of a composition of the invention can be applied directly to the scalp once or more times a day, conveniently for cycles lasting 2-3 months, alternating with rest periods.

The composition for topical application can be in solid, semi-solid or fluid form. Suitable formulations in solid form include creams, gels, pastes, ointments.

In other embodiments, the formulation for local administration is in the fluid form, for example in the form of lotions, gels, shampoos, suspensions, emulsions.

In the case of formulations in fluid or semi-fluid form, the biologically active components can be diluted in a carrier in the physiologically acceptable liquid form such as water, alcohol, hydroalcoholic or glycerin solution or mixed with other liquids suitable for local application.

In the form of a solution, suspension or dispersion, the composition of the invention may contain from about 1 to 99.9% of a liquid, such as water possibly mixed with an alcohol. In some embodiments, water is present in an amount in the range from 5 to 95%. In other embodiments, water is present in an amount in the range from 10 to 90% by weight.

A typical composition for topical use is a hydroalcoholic solution (water and ethanol) comprising the combination of maslinic acid and riboflavin dissolved therein.

By way of example, the compositions of the invention in liquid form can be prepared by dissolving the soluble components in water and the remaining components in alcohol, and then combining the various fractions under stirring. The resulting mixture can then be buffered to reach a pH range conveniently and preferably selected between 5 and 7 so as to be compatible with the pH of the scalp, and then be filtered and packaged in suitable containers such as bottles or vials.

In some embodiments, the composition of the invention is in the form of a lotion for external use.

Typically, the biologically active components, maslinic acid and riboflavin, of the compositions are formulated in dosage units. The dosage unit may contain from 0.1 to 1000 mg of active ingredient per dosage unit of the finished product for daily administration.

In some embodiments, the effective amounts for the formulation will depend on the severity of the disorder or condition to be treated. In some embodiments, the dose is in the range from 0.001% by weight to about 60% by weight of the formulation. According to some preferred aspects, the invention refers to compositions for topical use which are useful in the stimulation of hair follicles, with a consequent benefit in the treatment of hair growth disorders, such as alopecia, particularly in females. The treatment methods of the relevant population are also a feature of the invention. According to another aspect of the invention, a cosmetic treatment method is provided which comprises the application of the composition of the invention on the part to be treated, preferably on the scalp, of an effective amount of a composition of the type described above.

The amount administered and the frequency of administration of the composition will depend on the type and severity of the trichological condition to be treated. For example, for local uses, a cosmetically or pharmaceutically active amount of the composition of the invention is directly applied on the part to be treated, preferably on the scalp, one or more times a day, conveniently for cycles having a duration of 2-3 months alternating with rest periods.

The following examples are provided merely to illustrate the present invention and are not to be construed as limiting the scope of protection as shown by the accompanying claims.

### EXAMPLE 1

Composition for oral administration having the following formulation (not falling within scope of the invention as claimed) :

| **INGREDIENT** | **mg/tab** |
|---|---|
| Dry Extract (D.E.) SOY ISOFLAVONS @ 40% | 50.00-100.00 |
| VITAMIN D | 15.00-30.00 |
| ZINC | 15.00-20.00 |
| Borage Oil Powder | 10.00-15.00 |
| MANNITOL | 25.00-30.00 |
| CELLULOSE MICROCRYSTALLINE | 140.00-160.00 |
| Carminic Acid | 0,5-1.00 |
| CARBOXYMETHYLCELLULOSE | 2.00-3.00 |
| Demineralized water | |
| SODIUM CROSCARMELLOSE GF | 10.00-15.00 |
| VEGETABLE MAGNESIUM STEARATE | 3.00-5.00 |
| SILICA | 3.00-5.00 |
| Spermidine | 4.50-5.50 |
| Rutine | 1.00-2.00 |
| VITAMIN B2 | 15.00-20.00 |
| COPPER | 2,50-3.00 |
| Wakame Seaweed | 12.00-16.00 |
| MIXTURE NAMED NATUROLIVE CONTAINING MASLINIC ACID @ 30% BY WEIGHT | 50.00-60.00 |
| ANHYDROUS DICALCIUM PHOSPHATE (CAFOS) | 100.0000 |
| VEGETABLE MAGNESIUM STEARATE | 3.00-4.00 |

### EXAMPLE 2

Supplement for oral administration having the following formulation (not falling within the scope of the invention as claimed) :

| **INGREDIENT** | **mg/tab** |
|---|---|
| Dry Extract (D.E.) SOY ISOFLAVONS @ 40% | 50.00-100.00 |
| VITAMIN D | 15.00-30.00 |
| ZINC | 15.00-20.00 |
| Borage Oil Powder | 10.00-15.00 |
| MANNITOL | 25.00-30.00 |
| CELLULOSE MICROCRYSTALLINE | 140.00-160.00 |
| Carminic Acid | 0.5-1.00 |
| CARBOXYMETHYLCELLULOSE | 2.00-3.00 |
| Demineralized water | |
| SODIUM CROSCARMELLOSE GF | 10.00-15.00 |
| VEGETABLE MAGNESIUM STEARATE | 3.00-5.00 |
| SILICA | 3.00-5.00 |
| Spermidine | 4,50-5,50 |
| Rutine | 1.00-2.00 |
| VITAMIN B2 | 15.00-20.00 |
| COPPER | 2.50-3.00 |
| Alga Wakame | 12.00-16.00 |
| MIXTURE NAMED NATUROLIVE CONTAINING MASLINIC ACID @ 30% BY WEIGHT | 15.00-20.00 |
| ANHYDROUS DICALCIUM PHOSPHATE (CAFOS) | 100.0000 |
| VEGETABLE MAGNESIUM STEARATE | 3.00-4.00 |

### EXAMPLE 3

### Experimental Test.

In the context of this test, the maslinic acid used was contained in a mixture called *Naturolive* containing olive tree fruit extract (olive oil) with a terpene content of at least 75% by weight of which maslinic acid equal to 30% by weight (w/w) with respect to the weight of the composition, and mixture of maslinic acid and oleanolic acid greater than 75% by weight (w/w) with respect to the weight of the extract. Maslinic acid was combined 3:1 with riboflavin.

### MATERIALS AND METHODS

### Cell Culture

The PromoCell^{®} Human Follicle Dermal Papilla Cells (HFDPC) cell line was used. The following ingredients were used:

| **INTERNAL NAME** | **Maslinic acid (in a solid mixture of extracts from leaves and fruits of *Olea europea* containing maslinic acid at 30% by weight)** | **Riboflavin** | **Pure maslinic acid** |
|---|---|---|---|
| **STORAGE** | rt | rt | rt |
| **CONCENTRATION** | 18 µg/mL | 6 µg/mL | 18 µg/mL |

HFDPCs were isolated from the scalp dermis of healthy subjects.

HFDPCs (DP; PromoCell GmbH, Heidelberg, Germany, batch 325Z017.1 and 322Z030.1) were grown in base culture medium (Follicle Dermal Papilla Cell Growth Medium) (PromoCell GmbH) supplemented with 4 µL/mL of bovine pituitary gland (Bovine Pituitary Extract), 0.04 mL/mL fetal serum, 1 ng/mL recombinant human fibroblast growth factor, 5 µg/mL recombinant human insulin (PromoCell). Cells were cultured with 50 mg/mL primocin (Invivogen, Toulouse, France, #ant-pm-2) in a humidified incubator with 5% CO₂ at 37 °C.

The Follicle Dermal Papilla Cell Growth Medium contains all the growth factors and constituents necessary for the optimal growth of dermal papilla cells, as per the following table:

| **Component** | **Final Concentration** |
|---|---|
| Fetal Calf Serum | 0.04 mL/mL |
| Bovine Pituitary Extract | 0.004 mL/mL |
| Fibroblast Growth Factor (human recombinant) | 1 ng/mL |
| Insulin (human recombinant) | 5µg/mL |

### Controls

POSITIVE CONTROL: Cells treated in Follicle Dermal Papilla Cell Growth medium without serum (starved) under the same culture conditions.

NEGATIVE CONTROL: Untreated cells in Follicle Dermal Papilla Cell Growth medium under the same culture conditions.

### Reagents and instruments used

| **REAGENTS** | **SUPPLIER** |
|---|---|
| Human Follicle Dermal Papilla Cells (HFDPC) | Promocell PCC12071 |
| DetachKit-30. 330 ml | Promocell PCC41200 |
| Follicle Dermal Papilla Cell medium | Promocell PCC26501 |
| Cryo-SFM. 30 ml | Promocell PCC29910 |
| Tri reagent Solution | Thermo Fisher, AM9738 |
| Ethidium bromide solution (10 mg/mL, for molecular biology, aqueous solution) | SIGMA, E1510 |
| Gel Loading Buffer RNAse, none detected | SIGMA, G2526 |
| PRIME SCRIPT RT reagent kit (Perfect Real time) 200 rxn | TAKARA, RR037A |
| PreMix Ex Taq | TAKARA, RR039A |
| Chloroform | SIGMA, 366919-1L |
| Isopropanol | SIGMA, I9516-500 ml |
| TaqMan^{®} Gene Expression Assays for GAPDH Hs99999905_m1 | APPLIED BIOSYSTEMS, 4331182 |
| TaqMan^{®} Gene Expression Assays for PYGL Hs00958087_m1 | APPLIED BIOSYSTEMS, 4331182 |

| **EQUIPMENT** | **SUPPLIER** |
|---|---|
| Inverted Phase Contrast Microscope (Mod: DMIL) | Leica |
| Laminar flow cabinet (Mod: Gemini) + UV lamp with anti-reflex equipment | Sterile Manufacturing Division |
| HeraCell CO₂ incubator ( Mod:150 ADV) | Thermo Scientific |
| 15 L digital water bath from +5°C to + 100°C (Mod: bd1, BS-SWB2D) | Stuart |
| 85°C horizontal freezer ULT130, 120 L (Mod: Labfrost, MME-TE21140) | Elcold |
| Bürker counting chamber w/clamps (DI-DA-443/3) | Carlo Erba |
| Balance (Mod. AM100) | Mettler |
| RT PCR MX300P | Stratagene |
| Vortex | Arhos160-PBI International |

### RNA extraction, qPCR analysis

PYGL gene expression was studied by RT-PCR. The analysis was performed according to the method reported below (Gibson et al, 1996; Heid et al, 1996) using Taqman probes.

### a) Day 1: Cell seeding

When cells (HFDPC) reached approximately 80% confluence, they were detached with trypsin/EDTA and seeded at a density of 1×10⁶ cells/mL into 12-well plates and incubated at 37°C, 5% CO₂ (24h).

### b) Days 2-3: Treatment

Once 80% confluence was reached, the exhausted medium was removed, and the cells were treated for 24h with serum-free Follicle Dermal Papilla Cell Growth medium (starved) in the same culture conditions. Cells treated in complete culture medium served as a negative control.

After 24h, the cells were treated with the substances under evaluation:
#1: Maslinic acid (in solid mixture of olive tree leaf and fruit extracts containing maslinic acid at 30% by weight): 18 µg/mL
#2: Riboflavin 6 µg/mL
#3: Pure maslinic acid: 18 g/mL
#5: Maslinic acid (in solid mixture of olive tree leaf and fruit extracts containing maslinic acid at 30% by weight) 18 µg/mL + Riboflavin 6 µg/mL
#8: Maslinic acid (in solid mixture of olive leaf and fruit extracts containing maslinic acid at 30% by weight) 18 µg/mL+ Riboflavin 6 µg/mL

The pure substances were dissolved in DMSO (1000X stock solution) and diluted to the final concentration in Human Follicle Dermal Papilla Cells Growth medium. Positive and negative controls were included in the experiment.

The cells were incubated at 37°C, 5% CO₂ for 24h.

### c) RT-PCR

Gene expression of the various markers was evaluated by relative quantitative RT-PCR (quantitative reverse transcription-polymerase chain reaction-qRT-PCR). This analysis involved 3 sequential steps:
- extraction of total RNA;
- reverse transcription into cDNA;
- qRT-PCR.

Total RNA was extracted using the RNeasy mini kit according to the method described by Chomczynski and Mackey (6). At the end of the extraction, the extracted RNA was quantified using the QiaExpert instrument (Qiagen) and the concentrations, in µg/mL, of total RNA extracted at a wavelength of 260 nm were calculated.

Finally, the integrity of RNA (2 µg/mL) was assessed by an electrophoretic run on 1% agarose gel.

Total RNA was converted into cDNA (complementary DNA), using an enzyme capable of synthesizing a DNA molecule using an RNA strand as a template; this RNA dependent-DNA polymerase enzyme is called reverse transcriptase.

It binds to the 3' end of a single strand of RNA and synthesizes the cDNA strand using random primers and deoxynucleotide triphosphates (DNTP).

For this purpose, a commercial kit "PrimeScriptTM RT Reagent Kit (perfect Real Time)" (TakaraBiolnc., Japan) containing 5X PrimeScript Buffer (for real Time) PrimeScript RT Enzyme Mix1; OligodTPrimer; Random 6 weds; RNAse free dH₂O was used.

The extracted and quantified RNA was diluted to a concentration of 2 µg/mL and reverse transcribed into cDNA. A 10 µL Master Mix was prepared (containing 5X PrimeScript Buffer (for real Time); PrimeScript RT Enzyme Mix1; OligodTPrimer 50µM; Random 6 mers 100µM) to which 10 µL of RNA (2 µg/mL) were added. The samples were placed in a thermocycler (Stratagene Mx3000P Real Time PCR System, Agilent Technologies Italia S.p.A., Milan, Italy) and subjected to reverse transcription under the following conditions:
37°C for 15 minutes;
85°C for 5 seconds;
   4°C hold.

At the end of reverse transcription, 30 µL of DEPC water were added to the samples to obtain a final cDNA concentration of 40 ng/µL.

qRT-PCR represents a method of real-time amplification and quantification of the amplicons produced by monitoring the fluorescence emitted during the reaction. The TaqMan^{®} probe system (AppliedBiosystems) was used for RT-PCR amplification. The following TaqMan probes were used: Hs00958087_m1 (PYGL) and Hs99999905_m1 (GAPDH). GAPDH was used as a control gene (housekeeping).

The Taqman probe is a type of probe that enables the development of fluorescence as amplification progresses. A reporter (FAM^{™} fluorophore) is linked to its 5' end, while a quencher is linked to the 3' end. The proximity between the reporter and the quencher cancels the emission of the fluorescence signal. Fluorescence is detected only with the 5' exonuclease activity of the thermostable DNA polymerase (Taq polymerase) and the accumulation of the amplification products can be evaluated by the increase in fluorescence of the reporter which increases with each cycle.

For qRT-PCR a Master Mix was set up as follows:
- 10 µL of "2X Premix Ex Taq";
- 1 µL of "20× TaqMan Gene ExpressionAssays" (containing 2 primers and the fluorophore-labeled fluorescent probe FAM^{™});
- 0.4 µL of Rox II Passive Reference Dye;
- 5 µL of DEPC water.

4 µL of cDNA for the target gene and 1 µL of cDNA for the housekeeping gene were added to the Master Mix.

The amplification was carried out under the following conditions for 40 cycles:

| | | |
|---|---|---|
| • 95°C, | 30 sec | (Amplitaq activation); |
| • 95°C, | 5 sec | (Denaturation) |
| • 60°C, | 20 sec | (Annealing - extension); |

Each analysis was carried out in duplicate.

The data obtained were analyzed according to the 2^{-ΔΔCt} method and it was thus possible to calculate the relative expression values of the gene of interest, normalized with respect to the housekeeping gene and calibrated on the control sample (untreated cells): $ΔΔCt = Δ {Ct}_{target - housekeeping} \left(control\right) - Δ {Ct}_{target - housekeeping} \left(treated celles\right)$

The 2^{-ΔΔCt} was calculated assuming a 100% amplification efficiency.

### d) Statistical analysis

The statistical analysis was carried out using the software GraphPad Prism version 6.00 per Windows (GraphPad Software, La Jolla California USA, www.graphpad.com").

Using the Student's T test, the means and relative standard errors of all the formulations analyzed were compared with the positive control, using a 95% confidence interval.

### RESULTS

As reported in Figure 1, maslinic acid (18 µg/mL) was able to significantly inhibit (p<0.005) the expression of PYGL in dermal papilla cells. This inhibition was apparent both when maslinic acid was tested as such and in the solid mixture of olive leaf and fruit extracts containing maslinic acid at 30% by weight (Figure 1). Riboflavin (6 µg/mL) was also able to exert this significant effect (p<0.005) of inhibition of PYGL gene expression (Figure 1). Surprisingly, both the association of the solid mixture of olive leaf and fruit extracts containing maslinic acid at 30% by weight with riboflavin and of maslinic acid as such with riboflavin, exerted a synergistic effect and significantly (p<0.005) higher inhibition of PYGL gene expression, with respect to the positive control and individual components (Figure 1). The two activities resulted to be comparable.

## Claims

1. A non-therapeutic, cosmetic use of a composition for improving an appearance or aesthetic characteristic of a keratin structure selected from hair and skin appendages and/or for promoting its physiological growth in a mammal, wherein said composition comprises a combination of maslinic acid and riboflavin, and a physiologically acceptable excipient.

2. The use according to claim 1, wherein said keratin structure is selected from the group consisting of nails, hair, beard, eyelashes and eyebrows of a human being or hair of an animal being.

3. The use according to claim 1 or 2, wherein the maslinic acid is of natural origin and is in the form of an extract obtained by extraction from olive tree leaves and/or fruits, preferably from olive peel.

4. The use according to claim 3, wherein the maslinic acid is in the form of an extract obtained by extraction from olive tree leaves and/or fruits further comprising oleanolic acid.

5. The use according to any one of claims 1-4, wherein the composition is used by oral administration or topical application, preferably on the scalp.

6. A composition comprising a combination of maslinic acid and riboflavin, and a physiologically acceptable excipient, for use in the treatment of a disorder in the growth of a keratin structure selected from hair and skin appendages of a mammal.

7. The composition for use according to claim 6, wherein the keratin structure corresponds to hair of a human being.

8. The composition for use according to claim 6, wherein the keratin structure corresponds to skin appendages selected from the group consisting of nails, beard, eyelashes and eyebrows of a human being.

9. The composition for use according to any one of claim 6 to 8, wherein said disorder in the growth of a keratin structure is selected from the group consisting of alopecia, androgenetic alopecia, alopecia areata, ringworm, cicatricial alopecia and telogen effluvium.

10. The composition for use according to any one of claims 6-9, wherein the maslinic acid is of natural origin and is in the form of an extract obtained by extraction from olive tree leaves and/or fruits, preferably from olive peel.

11. The composition for use according to claim 10, wherein the maslinic acid is in the form of an extract obtained by extraction from olive tree leaves and/or fruits further comprising oleanolic acid.

12. The composition for use according to any one of claims 6-11, wherein the composition is used for oral administration or topical application.

## Patentansprüche

1. Nichttherapeutische, kosmetische Verwendung einer Zusammensetzung zur Verbesserung des Erscheinungsbildes oder einer ästhetischen Eigenschaft einer Keratinstruktur, ausgewählt aus Haar und Hautanhangsgebilden, und/oder zur Förderung ihres physiologischen Wachstums bei einem Säugetier, wobei die Zusammensetzung eine Kombination aus Maslinsäure und Riboflavin sowie einen physiologisch verträglichen Hilfsstoff umfasst.

2. Verwendung nach Anspruch 1, wobei die Keratinstruktur aus der Gruppe ausgewählt ist, bestehend aus Nägeln, Haar, Bart, Wimpern und Augenbrauen eines Menschen oder Haar eines Tieres.

3. Verwendung nach Anspruch 1 oder 2, wobei die Maslinsäure natürlichen Ursprungs ist und in Form eines Extrakts vorliegt, der durch Extraktion aus Olivenbaumblättern und/oder -früchten, vorzugsweise aus Olivenschalen, gewonnen wird.

4. Verwendung nach Anspruch 3, wobei die Maslinsäure in Form eines Extrakts vorliegt, der durch Extraktion aus Olivenbaumblättern und/oder -früchten gewonnen wird und ferner Oleanolsäure enthält.

5. Verwendung nach einem der Ansprüche 1-4, wobei die Zusammensetzung durch orale Verabreichung oder topische Anwendung, vorzugsweise auf der Kopfhaut, verwendet wird.

6. Zusammensetzung, umfassend eine Kombination aus Maslinsäure und Riboflavin sowie einen physiologisch verträglichen Hilfsstoff, zur Verwendung bei der Behandlung einer Wachstumsstörung einer Keratinstruktur, ausgewählt aus Haar und Hautanhangsgebilden eines Säugetiers.

7. Zusammensetzung zur Verwendung nach Anspruch 6, wobei die Keratinstruktur dem Haar eines Menschen entspricht.

8. Zusammensetzung zur Verwendung nach Anspruch 6, wobei die Keratinstruktur Hautanhangsgebilden entspricht, die aus der Gruppe ausgewählt sind, bestehend aus Nägeln, Bart, Wimpern und Augenbrauen eines Menschen.

9. Zusammensetzung zur Verwendung nach einem der Ansprüche 6 bis 8, wobei die Wachstumsstörung einer Keratinstruktur aus der Gruppe ausgewählt ist, die aus Alopezie, androgenetischer Alopezie, Alopecia areata, Ringelflechte, vernarbender Alopezie und telogenem Effluvium besteht.

10. Zusammensetzung zur Verwendung nach einem der Ansprüche 6-9, wobei die Maslinsäure natürlichen Ursprungs ist und in Form eines Extrakts vorliegt, der durch Extraktion aus Olivenbaumblättern und/oder -früchten, vorzugsweise aus Olivenschalen, gewonnen wird.

11. Zusammensetzung zur Verwendung nach Anspruch 10, wobei die Maslinsäure in Form eines Extrakts vorliegt, der durch Extraktion aus Olivenbaumblättern und/oder -früchten gewonnen wird und ferner Oleanolsäure enthält.

12. Zusammensetzung zur Verwendung nach einem der Ansprüche 6-11, wobei die Zusammensetzung zur oralen Verabreichung oder zur topischen Anwendung verwendet wird.

## Revendications

1. Utilisation cosmétique non thérapeutique d'une composition pour améliorer l'aspect ou une caractéristique esthétique d'une structure de kératine choisie parmi les cheveux et les phanères et/ou pour favoriser sa croissance physiologique chez un mammifère, dans laquelle ladite composition comprend une combinaison d'acide maslinique et de riboflavine, et un excipient physiologiquement acceptable.

2. Utilisation selon la revendication 1, dans laquelle ladite structure de kératine est choisie dans le groupe constitué par les ongles, les cheveux, la barbe, les cils et les sourcils d'un être humain ou les poils d'un être animal.

3. Utilisation selon la revendication 1 ou 2, dans laquelle l'acide maslinique est d'origine naturelle et se présente sous la forme d'un extrait obtenu par extraction à partir de feuilles et/ou de fruits d'olivier, de préférence à partir de peau d'olive.

4. Utilisation selon la revendication 3, dans laquelle l'acide maslinique est sous la forme d'un extrait obtenu par extraction à partir de feuilles et/ou de fruits d'olivier comprenant en outre de l'acide oléanolique.

5. Utilisation selon l'une quelconque des revendications 1 à 4, dans laquelle la composition est utilisée par administration orale ou application topique, de préférence sur le cuir chevelu.

6. Composition comprenant une combinaison d'acide maslinique et de riboflavine, et un excipient physiologiquement acceptable, pour son utilisation dans le traitement d'un trouble de la croissance d'une structure de kératine choisie parmi les cheveux et les phanères d'un mammifère.

7. Composition pour son utilisation selon la revendication 6, dans laquelle la structure de kératine correspond aux cheveux d'un être humain.

8. Composition pour son utilisation selon la revendication 6, dans laquelle la structure de kératine correspond à des phanères choisis dans le groupe constitué par les ongles, la barbe, les cils et les sourcils d'un être humain.

9. Composition pour son utilisation selon l'une quelconque des revendications 6 à 8, dans laquelle ledit trouble de la croissance d'une structure de kératine est choisi dans le groupe constitué par l'alopécie, l'alopécie androgénétique, l'alopécie areata, la teigne, l'alopécie cicatricielle et l'effluvium télogène.

10. Composition pour son utilisation selon l'une quelconque des revendications 6 à 9, dans laquelle l'acide maslinique est d'origine naturelle et se présente sous la forme d'un extrait obtenu par extraction à partir de feuilles et/ou de fruits d'olivier, de préférence à partir de peau d'olive.

11. Composition pour son utilisation selon la revendication 10, dans laquelle l'acide maslinique se présente sous la forme d'un extrait obtenu par extraction à partir de feuilles et/ou de fruits d'olivier comprenant en outre de l'acide oléanolique.

12. Composition pour son utilisation selon l'une quelconque des revendications 6 à 11, dans laquelle la composition est utilisée pour l'administration orale ou l'application topique.
